# EUROPEAN PATENT SPECIFICATION

(11) **EP 2 252 255 B1**
(45) Date of publication and mention of the grant of the patent: **01.08.2012**
(21) Application number: 08869164.7
(22) Date of filing: 09.12.2008
(51) Int. Cl.: A61K 8/19, A61K 8/44, A61K 8/49, A61K 8/64, A61K 8/23, A61K 8/26, A61K 8/27, A61Q 9/04

(54) **DEPILATORY KIT RESULTING IN REDUCED ODOR AND IRRITATION**
ENTHAARUNGSKIT MIT REDUZIERTER GERUCHSENTFALTUNG UND REIZUNG
DISPOSITIF DE DÉPILATION AVEC RÉDUCTION DE L'ODEUR ET DE L'IRRITATION

(30) Priority: 21.12.2007 US 15953
(43) Date of publication of application: 24.11.2010
(73) Proprietor: The Procter & Gamble Company, Cincinnati, OH 45202 (US)
(72) Inventor: BREYFOGEL, Laurie, Ellen, Milford Ohio 45150 (US); ZUKOWSKI, Joseph, Michael, Cincinnati Ohio 45249 (US); ELSBROCK, Robert, John, Cincinnati Ohio 45243 (US)
(74) Representative: Simpson, Kirsty Mairi
(86) International application number: PCT/IB2008/055181
(87) International publication number: WO 2009/083836

(56) References cited:
- WO-A1-2006/068276
- WO-A2-2007/046097
- CH-A- 290 140
- DE-A1- 3 828 709
- US-A1- 2006 034 874
- US-B1- 6 231 846
- K. Parfitt: "Martindale The complete drug reference, 32nd edition" 1999, the Pharmaceutical Press , London , XP002588156 ISBN: 0-85369-429 X , page 1096 column 2 - column 3
- "B.1.1.3 Zinkoxid" In: "Rote Liste 2007" 1 January 2007 (2007-01-01), Rote Liste Service GmbH , Frankfurt/Main , XP002588157 ISBN: 9783939192107 paragraph [85029] - paragraph [85036]

## Description

### FIELD OF THE INVENTION

This invention is related to a depilatory kit comprising a depilation composition and a neutralization composition.

### BACKGROUND OF THE INVENTION

It is common practice among much of the world's population to remove unwanted hair for cosmetic purposes. Women are especially desirous of removing unwanted hair on the face, underarms, legs and other body parts. Common mechanical depilation techniques include shaving, tweezing, and waxing. These mechanical depilation techniques each have unwanted disadvantages such as pain, noticeable stubble, or razor bumps (pseudofolliculitis barbae). Chemical depilatories are presently available in retail. Chemical depilatory compositions are convenient, because they destroy hair more deeply than shaving, and therefore can be used less often, and without the risk of cuts or abrasions. Most chemical depilatories presently available use mercaptans (i.e., thiols), particularly salts of thioglycolic acid, as the active depilation agent. Thioglycolate depilatories work by breaking down the disulfide bonds present in the cystine molecules of keratinous tissue such as found in the hair shaft.

Modern chemical depilatories usually include an aqueous phase comprising a thioglycolate salt (*e.g*., alkali or alkaline earth metal thioglycolates such as potassium or calcium thioglycolate). Chemical depilatories may comprise thioglycolic acid and an alkaline earth metal hydroxide such as calcium hydroxide and/or sodium hydroxide. Excess hydroxide may be used to maintain a high alkalinity of the depilatory (generally in the range upward of about pH 10 or, more commonly at about 12 to 13) which promotes the availability of free thioglycolate ion.

Although the thioglycolates are safe and effective, disadvantages are known. Thiols are very well known for their strong unpleasant and often appalling odor. This odor problem is further exacerbated when the depilatory product is targeted for facial hair and is used in proximity to the nose. Furthermore, significant irritation and inflammation can arise from the use of thioglycolate depilatories. The irritation is caused in part due to the high alkalinity and prolonged contact time (i.e., greater than 5 minutes) with the skin. Thioglycolate is known to be a contact allergen to persons having sensitive skin. This irritation prevents routine use of such compositions on the body, particularly on tender mucosal surfaces, and sensitive facial skin.

Various attempts have been made to address the problems associated with thiol depilatories. Limiting the contact time often is not an option since the thioglycolate needs sufficient time to penetrate the hair and cleave the disulfide bonds. Limiting contact time typically results in limited hair removal which is undesirable and defeats the purpose of the depilatory.

Fragrances are commonly used to mask the odor associated with thiol depilatories. Because thiols produce a strong unpleasant odor, a relatively high percentage of fragrance is often required to effectively mask the strong odor. Increased fragrance loading is not without drawbacks. Fragrances are a common skin irritant to many individuals which only worsens the irritation problem associated with thiol usage. Additionally, heavy scented products are disfavored by certain consumers.

Metal oxide compounds such as zinc oxide have been incorporated with thioglycolate depilatories as a means to reduce malodor. U.S. Patent No. 6,231846 found that thioglycolate-based hair treatment products containing zinc compounds, particularly zinc oxide, substantially reduced the production of methanethiol. However, zinc compounds such as zinc oxide may have an adverse effect on the efficacy of thioglycolate depilatories. It is the thioglycolate ion that cleaves the disulfide bonds present in the cystine molecules of keratin in hair. If zinc oxide is present in the depilatory, a portion of the thioglycolate ions may react with the zinc oxide to form zinc thioglycolate. As a result, the zinc oxide reduces the effectiveness of the depilatory by removing a portion of the thioglycolate ion that could be cleaving the disulfide bonds of the hair. Additional thioglycolate can be added to compensate but the additional thioglycolate further exacerbates the problems of odor and irritation.

In light of the problems described above, there is a continuing need for a chemical depilatory with reduced odor and which causes less skin irritation. As will become apparent from the following description, the present invention fulfills all of these needs.

### SUMMARY OF THE INVENTION

The present invention relates, in certain embodiments, to a depilatory kit comprising a depilatory composition comprising a sulfur-containing depilatory agent and a finishing composition comprising zinc oxide. In certain embodiments, the depilatory composition may further comprise a lactone such as 1,15-cyclopentadecanolide.

The present invention further relates, in other embodiments, to a facial depilatory kit comprising a wash-off depilatory composition and a leave-on finishing composition. The wash-off depilatory composition may comprise about 0.005% to about 10%, by weight of the depilatory composition, of a thioglycolate salt, and about 0.005% to about 3%, by weight of the depilatory composition, of 1,15-cyclopentadecanolide. The leave-on finishing composition may comprise about 0.01% to about 15%, by weight of the finishing composition a metal oxide selected from zinc oxide. In certain embodiments, the depilatory composition may further comprise a lactone such as 1,15-cyclopentadecanolide.

The present invention further relates to methods for using the depilatory kits described herein.

### DETAILED DESCRIPTION OF THE INVENTION

In all embodiments of the present invention, all percentages are by weight of the total composition, unless specifically stated otherwise. All ratios are weight ratios, unless specifically stated otherwise. The number of significant digits conveys neither a limitation on the indicated amounts nor on the accuracy of the measurements. Unless explicitly stated otherwise, all measurements are understood to be made at 25°C and at ambient conditions, where "ambient conditions" means conditions under about one atmosphere of pressure and at about 50% relative humidity. All such weights as they pertain to listed ingredients are based on the active level and do not include carriers or by-products that may be included in commercially available materials, unless otherwise specified.

"Dose" refers the amount of composition to be used during a single application.

"Unit-dose contain" refers to a container (*e.g*., vial, vessel, ampule, pouch, can, carton, canister, capsule, tube, substrate, wipe, pad, cup, blister pack, or like devices) containing a single dose of a skin care composition which is to be completely dispensed and applied.

"Bulk container" refers to a container containing a plurality of doses of a skin care composition.

"Leave-on" means a composition that is topically applied without washing off after a given length of time.

"Wash-off" means a composition that is topically applied by a user and then removed by the user after a period of time. The method of removal is not limited and can include, for example, rinsing with water or other liquid, wiping with a substrate, or scraping with an implement.

The depilatory kit of the present invention comprises a depilatory composition and a finishing composition. The depilatory care kit may be used for the removal of unwanted hair from the face, underarms, legs and other body part parts. As identified in the background, the problems the present depilatory kit solves (*e.g*., odor and irritation) make it particularly suitable for use on the face and in proximity to the nose.

The depilatory kit is not limited in construction or physical form. To preserve the efficacy of the depilatory composition, the depilatory composition and finishing composition may be housed such that they do not mix. This may be achieved by storing the depilatory composition and finishing composition in separate containers such as but not limited to a bottle, tube, vial, capsule, and the like. In other embodiments, the depilatory composition and finishing composition may be stored in separate compartments of a dual- or multi-compartmental container that allows for separate dispensing of the two compositions without mixing.

In certain embodiments, the depilatory kit may be in the form of a consumer unit. A "consumer unit" is a single entity for consumer sale that contains the components of the kit. The consumer unit may comprise several packages, boxes, or other like containers that are joined to form a single entity (*e.g*., several smaller packages contained within a larger container, several packages bound or adhered to for a single entity, etc.). For example, the kit may comprise a plurality of unit-dose container each having the depilatory composition and a bulk container having the finishing composition. Various configurations of containers within a consumer unit are envisioned.

The depilatory composition comprises a sulfur-containing depilatory agent. A "sulfur-containing depilatory agent" is a compound capable of removing or softening hair (such as by reacting with disulfide bonds of keratin) wherein such compound (i) contains one or more thiol groups or (ii) is a sulfide. Suitable thiol containing compounds include thioglycolic acid, thioglycolate salts (e.g., calcium, sodium, strontium, potassium, ammonium, lithium, magnesium, and the like), thioethylene glycol, thioglycerol, thioethanol, thioactic acid, thiosalicylic acid and salts thereof. Suitable of thiol-containing compounds may also include amino acids or their derivatives such as L-cysteine, D-cysteine, DL-cysteine, N-acetyl-L-cysteine, DL-homocysteine, N-carbamoyl cysteine, glutathion, and cysteamine, and salts and esters thereof (*e.g*., methyl and ethyl esters). Suitable examples of sulfides include calcium sulfide, sodium sulfide, potassium sulfide, lithium sulfide, and strontium sulfide. In certain embodiments, the sulfur-containing depilatory agent is a thioglycolate salt. In specific embodiments, the sulfur-containing depilatory agent is calcium thioglycolate.

The depilatory composition may comprise from about 0.005% to about 10% of the sulfur-containing depilatory agent. In other embodiments, the depilatory composition may comprise preferably from about 0.1% to about 5%, from about 1% to about 4%, or from about 2% to about 3.5% of the sulfur-containing depilatory agent. The concentration of the sulfur-containing depilatory agent may vary based upon the target area and the type of hair being depilated. Lower concentration of the sulfur-containing depilatory agent may be used when the target area involves sensitive skin such as around the face and pubic region or when depilating vellus hair. "Vellus hair" means a fine, short hair of less than 1 cm in length, containing little or no pigmentation. Higher concentrations of the sulfur-containing depilatory agent may be used when depilating terminal hair. "Terminal hair" means a coarse, pigmented, medullated hair which is longer than a vellus hair. Terminal hairs are typically seen on the scalp, eyebrows, and eyelashes, and include secondary sexual hair seen in the pubic region, abdomen, face and axillae.

The depilatory composition is ideally alkaline. In certain embodiments, the pH of the depilatory composition is between about 10 to about 13. It has been found that efficacy can be maintained at a pH of greater than 11.5 and irritation can be minimized at a pH less than 12.5.

The depilatory composition may further comprise a dermatologically acceptable carrier to enable the sulfur-containing depilatory agent to be delivered to the hair at an appropriate concentration. The carrier may be a solid, semi-solid or liquid. The carrier can be in a wide variety of forms. Non-limiting examples include simple solutions (water or oil based), emulsions, and solid forms (gels, sticks, flowable solids, amorphous materials). In certain embodiments, the dermatologically acceptable carrier is in the form of an emulsion. Emulsion may be generally classified as having a continuous aqueous phase (*e.g*., oil-in-water and water-in-oil-in-water) or a continuous oil phase (*e.g*., water-in-oil and oil-in-water-in-oil). The oil phase of the present invention may comprise silicone oils, non-silicone oils such as hydrocarbon oils, esters, ethers, and the like, and mixtures thereof. For example, emulsion carriers can include, but are not limited to, continuous water phase emulsions such as silicone-in-water, oil-in-water, and water-in-oil-in-water emulsion; and continuous oil phase emulsions such as water-in-oil and water-in-silicone emulsions, and oil-in-water-in-silicone emulsions. Emulsions may further comprise an emulsifier. The skin care composition may comprise from about 0.1% to about 10% or about 0.2% to about 5% of an emulsifier, based on the weight of the composition. Emulsifiers may be nonionic, anionic or cationic. Suitable emulsifiers are disclosed in, for example, U.S. Patent 3,755,560, U.S. Patent 4,421,769, and McCutcheon's Detergents and Emulsifiers, North American Edition, pages 317-324 (1986). Suitable emulsions may have a wide range of viscosities, depending on the desired product form.

The aqueous carriers typically include water. However, in other embodiments, the aqueous carriers may comprise components other than water (non-water components) such as alcohols, polyols, and other water miscible solvents. In one embodiment, the non-water component of the composition comprises a humectant such as glycerin and/or other polyols. However, it should be recognized that the carrier may be substantially (*i.e*., less than 1% water) or fully anhydrous.

The depilatory composition may further comprise a thickening agent as are well known in the art to provide suitable viscosity and rheological character. Exemplary thickening agents include gums, polysaccharides, carbomers, and polymers and copolymers of acrylic acid, acylanide, acryloyldimethyl taurate and their derivatives. It is also desirable to utilize gel network structures to thicken the depilatory composition. Suitable materials for preparing the gel network structures are well represented in the art and include fatty materials such as fatty alcohols (for example cetyl alcohol and stearyl alcohol) alone or used in conjunction with nonpolar oils such as paraffin or mineral oils. An appropriate emulsifier may also be used to form and stabilize the bilayer structure characteristic of gel network structures. In certain embodiments, the depilatory composition may have a suitable viscosity such that the composition does not readily flow during while in use. The viscosity of the depilatory composition is ideally greater than about 30,000 cps as measured on a Brookfield viscometer using a T-C bar spindle with a heliopath setting at 5 rpm at 25°C. In other embodiments, the viscosity of the depilatory composition is greater than about 70,000 cps.

In certain embodiments, the depilatory composition may comprise a lactone (*e.g*., cyclic ester) fragrance. Suitable lactone fragrances include, for example, 1,4-octanolide; 3-methyl-1,4-octanolide; 1,4-nonanolide; 1,4-decanolide; 8-decen-1,4-olide; 1,4-undecanolide; 1,4-dodecanolide; 1,5-decanolide; 1,5-dodecanolide; 1,15-cyclopentadecanolide; cis- and trans-11-pentadecen-1,15-olide; cis- and trans-12-pentadecen-1,15-olide; 1,16-hexadecanolide; 9-hexadecen-1,16-olide; 10-oxa-1,16-hexadecanolide; 11-oxa-1,16-hexadecanolide; 12-oxa-1,16-hexadecanolide; ethylene 1,12-dodecanedioate; ethylene 1,13-tridecane-dioate; coumarin; 2,3-dihydrocoumarin; and octahydrocoumarin. In a preferred embodiment, the depilatory composition comprises 1,15-cyclopentadecanolide (also commonly referred to as pentadecalactone.).

1,15-cyclopentadecanolide is known to have a musky, woody odor. Such an odor would generally not be preferred in a depilatory product that is, at least in certain embodiments, tailored toward female users. The inventors have found that 1,15-cyclopentadecanolide can effectively mask the thiol and sulfide compounds associated with sulfur-containing depilatory agent without producing a perceptible and disfavored musky, woody odor. The depilatory composition may comprise from about 0.005% to about 3% of the 1,15-cyclopentadecanolide and/or another lactone. In other embodiments, the depilatory composition may comprise from about 0.05% to about 0.75% or from about 0.1% to about 0.4% of 1,15-cyclopentadecanolide and/or another lactone. 1,15-cyclopentadecanolide can effectively mask the thiol and sulfide compounds associated with sulfur-containing depilatory agent without the need for other non-lactone fragrances. In certain embodiments, the depilatory composition comprises no more than about 10% of non-lactone fragrances. As used herein, "non-lactone fragrances" means a fragrance that is free of lactones. In other embodiments, the depilatory composition comprises no more than about 3% to about 5% of non-lactone fragrances. The depilatory composition may be free of non-lactone fragrances.

In certain embodiments, the depilatory composition comprises no more than about 10% of non-1,15-cyclopentadecanolide fragrances. As used herein, "non-1,15-cyclopentadecanolide fragrances" means a fragrance that is free of 1,15-cyclopentadecanolide. In other embodiments, the depilatory composition comprises no more than about 3% to about 5% of non-1,15-cyclopentadecanolide fragrances. The depilatory composition may be free of non-1,15-cyclopentadecanolide fragrances.

The finishing composition comprises a metal oxide selected from zinc oxide. The finishing composition may comprise from about 0.01% to about 15% of the metal oxide. In other embodiments, the depilatory composition may comprise from about 0.1% to about 5% or from about 0.5% to about 3%, of the metal oxide.

The finishing composition may further comprise a dermatologically acceptable carrier to enable the sulfur-containing depilatory agent to be delivered to the hair at an appropriate concentration. The carrier may be a solid, semi-solid or liquid. The carrier can be in a wide variety of forms. Non-limiting examples include simple solutions (water or oil based), emulsions, and solid forms (gels, sticks, flowable solids, amorphous materials). In certain embodiments, the dermatologically acceptable carrier is in the form of an emulsion. Emulsion may be generally classified as having a continuous aqueous phase (*e.g*., oil-in-water and water-in-oil-in-water) or a continuous oil phase (*e.g*., water-in-oil and oil-in-water-in-oil). The oil phase of the present invention may comprise silicone oils, non-silicone oils such as hydrocarbon oils, esters, ethers, and the like, and mixtures thereof. For example, emulsion carriers can include, but are not limited to, continuous water phase emulsions such as silicone-in-water, oil-in-water, and water-in-oil-in-water emulsion; and continuous oil phase emulsions such as water-in-oil and water-in-silicone emulsions, and oil-in-water-in-silicone emulsions. Emulsions may further comprise an emulsifier. The skin care composition may comprise from about 0.1% to about 10% or about 0.2% to about 5% of an emulsifier, based on the weight of the composition. Emulsifiers may be nonionic, anionic or cationic. Suitable emulsifiers are disclosed in, for example, U.S. Patent 3,755,560, U.S. Patent 4,421,769, and McCutcheon's Detergents and Emulsifiers, North American Edition, pages 317-324 (1986). Suitable emulsions may have a wide range of viscosities, depending on the desired product form.

The aqueous carriers typically include water. However, in other embodiments, the aqueous carriers may comprise components other than water (non-water components) such as alcohols, polyols, and other water miscible solvents. In one embodiment, the non-water component of the composition comprises a humectant such as glycerin and/or other polyols. However, it should be recognized that the carrier may be substantially (*i.e*., less than 1% water) or fully anhydrous.

The finishing composition may further comprise a safe and effective amount of one or more chronic skin care actives. As used herein, "chronic skin care active" means a compound useful for improving skin condition wherein the benefits are not realized at the time of application. The benefits of improved skin condition can include reduction of visibly and tactilely perceptible manifestations, as well as any macro- or micro-effects, due to keratinous tissue aging. Exemplary skin conditions include, but are not limited to, textural discontinuities such as wrinkles and coarse deep wrinkles, fine lines, skin lines, crevices, bumps, large pores, or unevenness; loss of skin elasticity; discoloration (including undereye circles); blotchiness; sallowness; hyperpigmented skin regions such as age spots and freckles; keratoses; abnormal differentiation; hyperkeratinization; elastosis; collagen breakdown, and other histological changes in the stratum corneum, dermis, epidermis, vascular system (*e.g*., telangiectasia or spider vessels), and underlying tissues (*e.g*., fat and/or muscle), especially those proximate to the skin. Usually the benefit of a chronic skin care active is achieved and/or perceived hours, days, or weeks after application. A chronic skin care active may require routine application in order for the benefit to be achieved and/or perceived. Chronic skin actives exclude skin conditioning agents and masking agents (*e.g*., titanium dioxide; iron oxides; matting particles including polyethylene, nylon, polymethylsilsesquioxane, and acrylic acid copolymers; interference pigments, or any other compound that provides an immediate change to skin appearance upon application).

Suitable chronic skin care actives include, but are not limited to, vitamins, peptides, sugar amines, sunscreens, oil control agents, tanning actives, anti-acne actives, desquamation actives, anti-cellulite actives, chelating agents, skin lightening agents, flavonoids, protease inhibitors, non-vitamin antioxidants and radical scavengers, hair growth regulators, anti-wrinkle actives, anti-atrophy actives, minerals, phytosterols and/or plant hormones, tyrosinase inhibitors, antiinflammatory agents, N-acyl amino acid compounds, antimicrobials, and antifungals. These chronic skin care actives are provided in further detail in U.S. application publication No. US2006/0275237A1 and US2004/ 0175347A1.

The finishing composition may further comprise one or more skin conditioning agents. Skin conditioning agents are useful for lubricating the skin, increasing the smoothness and suppleness of the skin, preventing or relieving dryness of the skin, hydrating the skin, and/or protecting the skin. The skin conditioning agent is preferably selected from the group consisting of emollients, humectants, and mixtures thereof. The skin conditioning agent is may be present in the finishing composition at a level of at least about 0.1%, about 1.0%, about 2.0%, or about 5.0% and at a level of no more than about 99%, about 50%, about 30%, or about 25%. Suitable emollients are designated as "Skin-Conditioning Agents - Emollient" in the Cosmetic, Toiletry, and Fragrance Association (CTFA) International Cosmetic Ingredient Dictionary and Handbook. 11th ed*.* Suitable humectants include polyols such as polyalkylene glycols, sorbitol, hydroxypropyl sorbitol, erythritol, threitol, pentaerythritol, xylitol, glucitol, mannitol, hexylene glycol, butylene glycol, hexane triol, glycerol, ethoxylated glycerol, propoxylated glycerol, and other compounds designated as "Skin-Conditioning Agents - Humectant" in the Cosmetic, Toiletry, and Fragrance Association (CTFA) International Cosmetic Ingredient Dictionory and Handbook. 11th ed*.*

The present application further describes a method of using the depilatory kits as embodied herein. In one embodiment, the depilatory kit may be provided to a user. The depilatory composition may be applied to a target surface such as the face, underarms, legs and other body part parts. The depilatory composition may be applied directly from the container onto the target area or may be placed on an intermediary (*e.g*., fingers, cotton swab, wash cloth, etc.) and then applied to the target area. A suitable dose may cover the target surface to a depth of approximately 1-2 mm.

The depilatory composition remains on the target surface for a prescribed contact time. Contact times generally span several minutes such as about 1 minute to about 15 minutes. The contact time may vary depending of the target surface and the hair type (*e.g*., vellus or terminal) being depilated. Shorter contact times (e.g., about 1 minute to about 8 minutes) may be appropriate when the depilatory composition is used in a sensitive target area or when depilating primarily vellus hair. Longer contact times (e.g., about 8 minutes to 15 minutes) may be used when depilating terminal hair. Contact times are selected to achieve satisfactory depilation while minimizing skin irritation. As the face is more sensitive and on the female face typically supports hairs of smaller diameter, shorter contact times (*e.g*., about 3 minutes to about 8 minutes) may be appropriate for depilation in this area and for women.

After the prescribed contact time, the depilatory composition may be removed along with the softened or disintegrated hair. The depilatory may be removed by a suitable method such as rinsing with water or another dermatologically-acceptable liquid, washing with water and a mild cleanser, wiping with a substrate such as a wash cloth, or scraping with an implement such as a blade-less razor. The removal step serves to remove the hair from the target area and to remove excess sulfur-containing depilatory agent from the target area. A thorough removal followed by a splash rinse can limit the amount of sulfur-containing depilatory agent left on the target area which may cause lingering odor and irritation issues.

The finishing composition may be applied to a target surface. The finishing composition may be applied such that it overlaps the area on which the depilatory composition was applied. The finishing composition may be applied directly from the container onto the target area or may be placed on an intermediary (*e.g*., fingers, cotton swab, wash cloth, etc.) and then applied to the target area. A suitable dose is from about 1 mg/cm² to about 3 mg/cm². The dose may be altered to meet the personal preferences of the user. The finishing composition may be applied in a timely manner after removal of the depilatory composition such as seconds or a few minutes after removal of the depilatory composition. The finishing composition may be a wash-off composition or a leave-on composition. Regardless of form, the finishing composition should remain on the skin a sufficient amount of time to allow the metal salt such as a metal oxide to complex with any remaining sulfur-containing depilatory agent remaining on the target area. It is believed that application of the metal salt such as a metal oxide (and zinc oxide in particular) reduces lingering odor and irritation caused by residual sulfur-containing depilatory agent. The metal oxide may be provided via the finishing composition after application and removal of the depilation composition. This allows the metal oxide to complex with the remaining sulfur-containing depilatory age while not adversely impacting the efficacy of the depilation which is complete.

In certain embodiments, the finishing composition is a leave-on composition. A leave-on finishing composition is particularly suitable when chronic skin care actives and/or skin conditioning agents are used. The increased contact time of a leave-on product generally improved the efficacy of most chronic skin care actives and/or skin conditioning agents. Furthermore, a leave-on product allows the metal oxide continue complexing with residual sulfur-containing depilatory agent.

In certain embodiments, a further step of reapplication of the finishing composition may be included in the method. In these embodiments, the depilatory composition has been applied and removed and at least one application of the finishing composition has occurred. The finishing composition may be periodically applied. Periodic reapplication is particularly suitable when chronic skin care actives and/or skin conditioning agents are used. The periodic application generally improves the efficacy of most chronic skin care actives or skin conditioning agents. The period of application can vary and includes daily application and multiple applications per day (*e.g*., at morning and night).

The method may be repeated as needed by the user. A typical period of use may be weekly; however, the hair growth needs of any particular user may require more or less frequent application.

### Examples

The following formulations are non-limiting examples of suitable depilatory and finishing compositions that may be paired to yield the abovementioned depilatory kit. Where applicable, ingredients are identified by the CTFA name. While particular embodiments of the subject invention have been described, it will be obvious to those skilled in the art that various changes and modifications to the subject invention can be made without departing from the spirit and scope of the invention.

**Depilatory Composition Examples:**

| **Example #** | | D1 | D2 | D3 * | D4 | D5 | D6 | D7 | D8 |
|---|---|---|---|---|---|---|---|---|---|
| **Ingredients** | | % Wt | % Wt | % Wt | % Wt | % Wt | % Wt | % Wt | % Wt |
| | **Phase A: Water Phase** | | | | | | | | |
| A1 | Water | qs | qs | qs | qs | qs | qs | qs | qs |
| A2 | **Calcium Hydroxide** | 0.10 | 0.10 | 0.10 | 0.10 | 0.10 | 0.10 | 0.10 | 0.10 |
| | | | | | | | | | |

| | **Phase B: Oil Phase** | | | | | | | | |
|---|---|---|---|---|---|---|---|---|---|
| B1 | Mineral Oil | 10.0 | 10.0 | 10.0 | 10.0 | 10.0 | 10.0 | 10.0 | 10.0 |
| B2 | Stearyl Alcohol | 5.00 | 5.00 | 5.00 | 5.00 | 5.00 | 5.00 | 5.00 | 5.00 |
| B3 | Cetyl Alcohol | 5.00 | 5.00 | 5.00 | 5.00 | 5.00 | 5.00 | 5.00 | 5.00 |
| B4 | Brij 721*¹ | 2.00 | 2.00 | 2.00 | 2.00 | 2.00 | 2.00 | 2.00 | 2.00 |
| | | | | | | | | | |

| | **Phase C: Others** | | | | | | | | |
|---|---|---|---|---|---|---|---|---|---|
| C1 | Calcium Thioglycolate Trihvdrate | 1.40 | 2.90 | 4.30 | 5.70 | 4.30 | 4.30 | --- | --- |
| C2 | Potassium Thioglycolate | --- | --- | --- | --- | --- | --- | 2.00 | 3.00 |
| C3 | Pentadecalactone | 0.12 | 0.15 | 0.20 | 0.25 | 0.25 | 0.25 | 0.20 | 0.25 |
| C4 | Fragrance | 0.25 | 0.25 | 0.25 | 0.25 | 0.15 | --- | 0.25 | 0.25 |

| | | | | | | | | | |
|---|---|---|---|---|---|---|---|---|---|
| * 1 - Steareth-21 available from Uniqema Americas, New Castle, DE. Note - Example D3 yields a viscosity of about 80,000 cps as measured according to the method described above. | | | | | | | | | |

### Preparation of Depilatory Composition Examples

### I. Phase A preparation:

1. Into suitable container, add A1-A2. Stir this mixture using a propeller mixer and heat to 75 °C until it is cleared and homogeneous.

### II. Phase B preparation:

1. Into suitable container, add B1-B4. Stir this mixture using a propeller mixer and heat to 75 °C until it is cleared and homogeneous.

### III. Final Steps:

1. With the phases at 75 °C, add phase B to A and mix the batch for 10 min using a propeller mixer. Mill the batch for 5 minutes using TK-50 at 8000 rpm.
2. Cool to 30 °C with mixing. Add C1-C4 to the batch one at a time by mixing with a propeller and/or anchor blade. Mix for 15 minutes or until homogenous and smooth.
3. Transfer the batch to a storage container.

### Finishing Composition Examples:

| **Example #** | | F1* | | F4 |
|---|---|---|---|---|
| **Ingredients** | | % Wt | | % Wt |
| | **Phase A: Water Phase** | | | |
| A1 | Water | qs | | qs |
| A2 | Glycerin | 10.00 | | 7.00 |
| A3 | Panthenol | 1.00 | | 0.50 |
| A4 | Niacinamide | - | | 3.50 |
| A5 | Sodium Ascorbyl Phosphate | - | | 0.10 |
| A6 | GLW65TAP | - | | 0.70 |
| A7 | Disodium EDTA | - | | 0.10 |
| | | | | |
| | **Phase B: Oil Phase** | | | |
| B1 | Cetyl Alcohol | 3.75 | | 0.72 |
| B2 | Stearyl Alcohol | 3.75 | | 0.48 |
| B3 | Brij 721 | 0.70 | | - |
| B4 | Brij 72^{*2} | 0.30 | | - |
| B5 | Petrolatum | 2.00 | | - |
| B6 | Dow Corning 2503^{*3} | 1.50 | | - |
| B7 | Isoproypl Isostearate | 2.00 | | 13.50 |
| B8 | Isohexadecane | - | | 3.00 |
| B9 | Vitamin E Acetate | - | | 0.60 |
| B10 | Sefa Cottonate | - | | 0.67 |
| B11 | Ethyl Paraben | - | | 0.20 |
| B12 | Propyl Paraben | - | | 0.10 |
| B13 | Mryj 59^{*4} | - | | 0.20 |
| B14 | BHT | - | | 0.05 |
| | | | | |
| | **Phase C: ZnO Premix** | | | |
| C1 | Zinc Oxide | 2.00 | | 2.00 |
| C2 | Aluminum Oxide | - | | - |
| C3 | Magnesium Oxide | - | | - |
| C4 | Water | 4.00 | | 4.00 |
| | | | | |
| | **Phase D: Others** | | | |
| D1 | Sepigel 305 ^{*5} | - | | 3.00 |
| D2 | Dow Corning 1503 ^{*6} | 2.00 | | 2.00 |
| D3 | Benzyl Alcohol | - | | 0.50 |
| D4 | Glydant Plus Liquid ^{*7} | 0.3 | | - |
| D5 | Fragrance | - | | 0.20 |

| | | | | |
|---|---|---|---|---|
| *2 - Steareth-2 available from Uniqema Americas, New Castle, DE. *3 - Stearyl Dimethicone available from the Dow Coming Corp., Midland, MI. *4 - PEG-100 available from Uniqema Americas, New Castle, DE. *5 - Acrylamide copolymer, C13-14 Isoparaffin, and Laureth-7 mixture available from SEPPIC Inc., Fairfield, NJ. *6 - Dimethicone and dimethiconol mixture available from the Dow Coming Corp., Midland, MI. *7 - DMDM Hydantoin available from Lonza, Inc., Allendale, NJ. Note - Example F1 yields a viscosity of about 80,000 cps as measured according to the method described above. | | | | |

### Preparation of Finishing Composition Examples

### 1. Phase A preparation:

1. Into suitable container, add A1-A7. Mix this mixture using a propeller mixer and heat to 75 °C until it is cleared and homogeneous.

### II. Phase B preparation:

1. Into suitable container, add B1-B14. Mix this mixture using a propeller mixer and heat to 75 °C until it is cleared and homogeneous.

### III. Phase C preparation:

1. Into a suitable container, add C1-C4. Mix this mixture using a propeller mixer to create a homogeneous slurry.

### IV. Final Steps:

1. Add phase B to A and mix the batch for 10 minutes using a propeller mixer. Mill the batch for 5 mins using TK-50 at 8000 rpm.
2. Cool to 30 °C with mixing via a propeller blade. Keeping the batch temperature between 45 and 40 °C, add phase C and D1-D5 to the batch one at a time by mixing with a propeller and/or anchor blade. Mix for 15 minutes or until homogenous and smooth.
3. Transfer the batch to a storage temperature.

### Comparative Study

A depilatory kit comprising a depilatory composition and a finishing composition has been found to noticeably reduce the irritation perceived by users when the kit is used for facial depilation. A study was performed comparing a depilatory kit comprising a depilatory composition (Example D2) and a finishing composition (Example F1) versus a depilatory composition (Example D2) alone. In both legs of the study, the application and removal of the depilatory composition was similar. Panelists applied the delipatory composition by hand to the face (avoiding the mouth, nostrils, and eyes). The depilatory composition remained on the face for about 8 minutes. The depilatory composition was removed using a wet washcloth followed by a water splash rinse and towel drying. In the leg using the depilatory kit, a finishing composition was applied at a dose of about 2.0 mg/cm².

The panelists answered various questions about irritation with answers being indexed according to a qualitative scale (e.g., In response to the statements "Product is not irritating to my skin" and "Product is not irritating to my eyes" panelists selected an appropriate response from the following choices: not at all=0, not very effective=25, somewhat effective=50, very effective=75, extremely effective=100). Answers were statistically analyzed and a mean score provided (p=0.05). Provided below are the results of two questions relating to the functionality or benefit of the Compositions.

| **Question** | **Depilatory Composition & Finishing Composition** | **Depilatory Composition** |
|---|---|---|
| Did not irritate skin | 52 | 42 |
| Did not irritate eyes | 64 | 56 |

The panelists' answers suggest the finishing composition with the metal oxide (*e.g*., zinc oxide) reduces the perceived irritation caused by the depilatory composition.

## Claims

1. A depilatory kit comprising:
a) a depilatory composition comprising a sulfur-containing depilatory agent: and
b) a finishing composition comprising a metal oxide selected from zinc oxide.

2. The depilatory kit of Claim 1 wherein the sulfur-containing depilatory agent is thioglycolic acid or a thioglycolate salt, preferably the sulfur-containing depilatory agent is calcium thioglycolate, potassium thioglycolate, or sodium thioglycolate

3. The depilatory kit according to any one of the preceding claims wherein the depilatory compositions has a viscosity of greater than 30,000 mPa.s, preferably greater than 70,000 mPa.s as measured on a Brookfield viscometer using a T-C bar spindle with a heliopath setting at 5 rpm at 25°C.

4. The depilatory kit according to any one of the preceding claims wherein the depilatory composition comprises 0.005% to about 1.0%, by weight of the composition, of the sulfur-containing depilatory agent.

5. The depilatory kit according to any one of the preceding claims wherein the depilatory composition further comprises a lactone, preferably 1,15-cyclopentadecanolide.

6. The depilatory kit according to any one of the preceding claims wherein the finishing composition comprise 0.01% to 15%, preferably 0.5% to about 3%, by weight of the composition, of the natal oxide.

7. The depilatory kit according to any one of the preceding claims wherein the Finishing composition further comprises a skin conditioning agent or a skin care active.

8. The depilatory kit according to any one of the preceding claims wherein the depilatory composition comprises no more than 10%, preferably no more than 1%, of a non-lactone fragrance.

9. The depilatory kit according to any one of the preceding claims wherein the depilatory composition comprises no more than 10%, preferably no more than 1%, of a non-1,15-cyclopentadecanolide fragrance.

10. The depilatory kit according to any one of the preceding claims wherein the depilatory composition is a wash-off composition and the finishing composition is a leave-on composition.

## Patentansprüche

1. Enthaarungsset, umfassend:
a) eine Enthaarungszusammensetzung, umfassend ein schwefelhaltiges Enthaarungsmittel; und
b) eine Endbearbeitungszusammensetzung, umfassend ein Metalloxid, das ausgewählt ist aus Zinkoxid.

2. Enthaarungsset nach Anspruch 1, wobei das schwefelhaltige Enthaarungsmittel Thioglycolsäure oder ein Thioglycolatsalz ist, wobei das schwefelhaltige Enthaarungsmittel vorzugsweise Kalziumthioglycolat, Kaliumthioglycolat oder Natriumthioglycolat ist.

3. Enthaarungsset nach einem der vorstehenden Ansprüche, wobei die Enthaarungszusammensetzung eine Viskosität von mehr als 30.000 mPa.s, vorzugsweise mehr als 70.000 mPa.s, aufweist, wie auf einem Brookfield-Viskosimeter unter Verwendung einer T-C Stab-Spindel mit einer Heliopath-Einstellung bei 5 U/min bei 25 °C gemessen.

4. Enthaarungsset nach einem der vorstehenden Ansprüche, wobei die Enthaarungszusammensetzung zu 0,005 Gew.-% bis etwa 10 Gew.-% der Zusammensetzung das schwefelhaltige Enthaarungsmittel umfasst.

5. Enthaarungsset nach einem der vorstehenden Ansprüche, wobei die Enthaarungszusammensetzung ferner ein Lacton, vorzugsweise 1,15-Cyclopentadecanolid umfasst.

6. Enthaarungsset nach einem der vorstehenden Ansprüche, wobei die Endbearbeitungszusammensetzung zu 0,01 Gew.-% bis 15 Gew.-%, vorzugsweise 0,5 Gew.-% bis etwa 3 Gew.-% der Zusammensetzung das Metalloxid umfasst.

7. Enthaarungsset nach einem der vorstehenden Ansprüche, wobei die Endbearbeitungszusammensetzung ferner ein Hautpflege-Konditioniermittel oder einen Hautpflegewirkstoff umfasst.

8. Enthaarungsset nach einem der vorstehenden Ansprüche, wobei die Enthaarungszusammensetzung zu nicht mehr als 10 %, vorzugsweise nicht mehr als 1 % einen Nicht-Lacton-Duftstoff umfasst.

9. Enthaarungsset nach einem der vorstehenden Ansprüche, wobei die Enthaarungszusammensetzung zu nicht mehr als 10 %, vorzugsweise nicht mehr als 1 % einen Nicht-1,15-Cyclopentadecanolid-Duftstoff umfasst.

10. Enthaarungsset nach einem der vorstehenden Ansprüche, wobei die Enthaarungszusammensetzung eine Zusammensetzung ist, die zum Abwaschen von der Haut bestimmt ist, und die Endbearbeitungszusammensetzung eine Zusammensetzung ist, die zum Belassen auf der Haut bestimmt ist.

## Revendications

1. Trousse dépilatoire comprenant :
a) une composition dépilatoire comprenant un agent dépilatoire contenant du soufre ; et
b) une composition de finition comprenant un oxyde métallique choisi parmi l'oxyde de zinc.

2. Trousse dépilatoire selon la revendication 1, dans laquelle l'agent dépilatoire contenant du soufre est de l'acide thioglycolique ou un sel thioglycolate, de préférence, l'agent dépilatoire contenant du soufre est du thioglycolate de calcium, du thioglycolate de potassium, ou du thioglycolate de sodium.

3. Trousse dépilatoire selon l'une quelconque des revendications précédentes, dans laquelle la composition dépilatoire a une viscosité supérieure à 30 000 mPa.s, de préférence, supérieure à 70 000 mPa.s, telle que mesurée sur un viscosimètre Brookfield en utilisant un mobile cylindrique à barre T-C avec un réglage Heliopath à 5 tr/min à 25 °C.

4. Trousse dépilatoire selon l'une quelconque des revendications précédentes, dans laquelle la composition dépilatoire comprend 0,005 % à environ 10 % en poids de la composition, de l'agent dépilatoire contenant du soufre.

5. Trousse dépilatoire selon l'une quelconque des revendications précédentes, dans laquelle la composition dépilatoire comprend en outre une lactone, de préférence du 1,15-cyclopentadécanolide.

6. Trousse dépilatoire selon l'une quelconque des revendications précédentes, dans laquelle la composition de finition comprend de 0,01 % à 15 %, de préférence de 0,5 % à environ 3 % en poids de la composition, de l'oxyde métallique.

7. Trousse dépilatoire selon l'une quelconque des revendications précédentes, dans laquelle la composition de finition comprend en outre un agent de conditionnement pour le soin de la peau ou un principe actif pour le soin de la peau.

8. Trousse dépilatoire selon l'une quelconque des revendications précédentes, dans laquelle la composition dépilatoire ne comprend pas plus de 10 %, de préférence pas plus de 1 %, d'un parfum différent d'une lactone.

9. Trousse dépilatoire selon l'une quelconque des revendications précédentes, dans laquelle la composition dépilatoire ne comprend pas plus de 10 %, de préférence pas plus de 1 %, d'un parfum différent du 1,15-cyclopentadécanolide.

10. Trousse dépilatoire selon l'une quelconque des revendications précédentes, dans laquelle la composition dépilatoire est une composition à éliminer par lavage et la composition de finition est une composition à laisser.
